(19) Europäisches Patentamt

European Patent Office

Office européen des brevets

(11) Veröffentlichungsnummer: **0 215 332**

**A2**

(12) # EUROPÄISCHE PATENTANMELDUNG

(21) Anmeldenummer: 86111611.9

(22) Anmeldetag: 22.08.86

(51) Int. Cl.⁴: **G01N 27/62**

(30) Priorität: 16.09.85 DD 280626

(43) Veröffentlichungstag der Anmeldung:
25.03.87 Patentblatt 87/13

(84) Benannte Vertragsstaaten:
AT CH DE FR GB IT LI NL SE

(71) Anmelder: **Drägerwerk Aktiengesellschaft**
**Moislinger Allee 53-55**
**D-2400 Lübeck 1(DE)**
Anmelder: **Akademie der Wissenschaften der**
**DDR**
**Otto-Nuschke-Strasse 22/23**
**DDR-1086 Berlin(DD)**

(72) Erfinder: **Grosse, Hans-Jörg, Dr. sc. nat.**
**Dipl.-phys.**
**Gomeniusstrasse 34**
**DDR-7050 Leipzig(DD)**
Erfinder: **Nietzschmann, Helga**
**Strelitzer Strasse 15**
**DDR-7022 Leipzig(DD)**
Erfinder: **Merten, Hartmut, Dipl.-Chem.**
**Am Markt 13**
**DDR-8090 Dresden(DD)**
Erfinder: **Plewinski, Klaus, Dipl.-Chem.**
**Hans-Grundig-Strasse 30**
**DDR-8019 Dresden(DD)**

(74) Vertreter: **Patentanwälte Beetz sen. - Beetz**
**jun. Timpe - Siegfried - Schmitt-Fumian**
**Steinsdorfstrasse 10**
**D-8000 München 22(DE)**

(54) Verfahren zur Bestimmung von Diboran in Luft.

(57) Diboran wird mit hoher Empfindlichkeit (ppb-Bereich) und Selektivität in Luft nachgewiesen. Die Erfindung ist damit für die Überwachung von Arbeitsplätzen und -räumen, der Emission von Anlage usw. geeignet. Der Nachweis beruht auf der Aerosolionisationsgasanalyse und besteht darin, daß zusätzlich zum Amin dem Luftstrom ein Reagens zugesetzt wird, das nicht mit dem Amin, aber mit dem Diboran zu einer mit dem Amin Aerosole bildenden Verbindung reagiert. Die durch diese Reaktion gewonnenen Aerosole bilden dabei den Hauptmeßeffekt. Bei Verwendung von $SO_2$ in Konzentrationen $\geq$ 3,5 ppm werden Störungen durch den $SO_2$-Gehalt der Luft ausgeschaltet.

## Verfahren zur Bestimmung von Diboran in Luft

### Anwendungsgebiet der Erfindung

Die Erfindung betrifft ein Verfahren zum hochempfindlichen Nachweis von Diboran in Luft - (ppb-Bereich) mit hoher Selektivität und Geschwindigkeit. Sie ist damit für die Überwachung von Arbeitsplätzen und -räumen, z.B. in der Mikroelektronik bei Gasphasendosierprozessen, bzw. der Emission von Anlagen sowie die Dichtheitskontrolle von Anlagen und ähnlichem geeignet.

### Charakteristik der bekannten technischen Lösungen

Für den Nachweis von Diboran werden verschiedene Verfahren eingesetzt. So gibt es Geräte, die auf dem Prinzip der Colorimetrie beruhen. Diese Geräte haben einen Nachweisbereich von 0 bis 87 ppb, sind also empfindlich und auch selektiv, haben aber eine lange Anreicherungszeit (ca. 1 Stunde). Sie sind damit für Havariefälle nicht geeignet. Außerdem sind die Bänder nur begrenzt lagerfähig; der Bedienungs-und Wartungsaufwand ist hoch.

Ein bedeutend schnelleres Verfahren beruht auf der dispersiven Infrarotspektrometrie (z.B. die Geräteserie MIRAN der Firma Foxboro Analytical, USA). Eine Messung dauert nur etwa 10 Sekunden, die Nachweisgrenze liegt aber nur bei 100 ppb, so daß viele Einsatzgebiete herausfallen.

Auch die Chemilumineszenz wird für den Nachweis von Diboran genutzt (z.B. FR 2 506 459). Die bei der Reaktion von Diboran mit Ozon entstehende Strahlung wird mit einem Sekundärelektronenvervielfacher gemessen. Auch dieses Verfahren ist schnell, die Empfindlichkeit jedoch gering. Die Nachweisgrenze liegt bei 2 ppm.

Schließlich besteht auch die Möglichkeit, die Aerosolionisationsgasanalyse für den Nachweis von Diboran einzusetzen. Ein radioaktives Präparat erzeugt in einer Ionisationskammer einen Ionisationsstrom, der sich bei Anwesenheit von Aerosolen ändert, wobei diese Änderung der Aerosolkonzentration proportional ist. Im Falle des Diborans wird die Reaktion mit einem Amin ausgenutzt, die zu gut nachweisbaren Aerosolen führt. Während das Verfahren ausreichend schnell arbeitet, genügt auch hier die Empfindlichkeit nicht. Die Nachweisgrenze liegt bei 150 ppb. Außerdem bewirkt der Schwefeldioxidgehalt der Luft eine Störung.

### Ziel der Erfindung

Das Ziel der Erfindung ist der schnelle, selektive und hochempfindliche Nachweis von Diboran in Luft.

### Darlegung des Wesens der Erfindung

Der Erfindung liegt die Aufgabe zugrunde, die Aerosolionisationsgasanalyse für Diboran so zu verändern, daß die Nachweisgrenze um mehr als eine Größenordnung verbessert und gegebenenfalls der störende Einfluß des $SO_2$ der Luft ausgeschaltet wird.

Das erfindungsgemäße Verfahren besteht darin, daß zusätzlich zum Amin (Konzentration ≥ 50ppm) dem Luftstrom ein Reagens zugesetzt wird, das nicht oder nur in vernachlässigbar geringem Maße mit dem Amin Aerosole bildet, aber mit dem Diboran zu Verbindungen reagiert, die mit dem Amin gut nachweisbare Aerosole bilden. Die durch diese Reaktion gewonnenen Aerosole bilden dabei den Hauptmeßeffekt (neben dem Meßeffekt, der durch die direkte Reaktion des Diboran mit dem Amin bewirkt wird).

Besonders günstig ist die Verwendung von Schwefeldioxid in Konzentrationen ≥ 3,5 ppm, weil dann das in der Luft enthaltene $SO_2$ keinen bzw. nur einen vernachlässigbar geringen Einfluß -uf die Messung besitzt ($SO_2$ reagiert kaum mit Amin).

Zur Beseitigung von Querempfindlichkeiten gegen stark sauer reagierende Komponenten in der Luft (z.B. $NO_2$, HCl) wird Zinkgranulat verwendet, das zwar das Diboran passieren läßt, stark sauer reagierende Stoffe aber durch chemische Reaktion beseitigt. Bei der Zuführung dès Reagens' in den zu analysierenden Luftstrom ist darauf zu achten, daß das vor der Zugabe des Amins erfolgen muß, damit zunächst die Reaktion des Diborans mit dem Reagens ablaufen kann. Um das zu erreichen, gibt es verschiedene Möglichkeiten: so kann das Reagens vor der Ionisationskammer in den Luftstrom, das Amin aber direkt in die Ionisationskammer dosiert werden, oder vom Luftstrom wird ein Teilstrom abgezweigt, in den das Reagens gegeben und dieser dann wieder mit dem Hauptstrom vereinigt wird, während das Amin wiederum in die Ionisationskammer gegeben wird. Es ist aber auch möglich, vom Luftstrom zwei Teilströme abzuzweigen, in die das Reagens bzw. das Amin dosiert werden. Diese Teilströme werden nacheinander wieder mit dem Hauptstrom vereint.

Die Zuführung des Reagens kann auf unterschiedliche, aber bekannte Art und Weise erfolgen. Im Falle der Verwendung von SO₂ ist folgende Variante empfehlenswert: durch Zersetzung von Na₂S₂O₅-Lösung wird SO₂ gewonnen, das durch eine geeignete Folie (z.B. Polyethylen) in definierter Konzentration in den Luftstrom permeiert. Das hat den Vorteil, daß im Meßvorgang selbst keine Flüssigkeiten benötigt werden.

Ausführungsbeispiel

Die zur Durchführung des erfindungsgemäßen Verfahrens eingesetzte Vorrichtung besteht aus einer Ionisationskammer mit eingebauter Strahlungsquelle, Einrichtungen zur Gasflußerzeugung, -regulierung und -anzeige, Meßwerterfassung und -verarbeitung, Vorratsgefäßen für Amin und Na₂S₂O₅-Lösung, Gasleitungen und Gasteilern. Die Bestimmung von Diboran in Luft wird wie folgt vorgenommen: mittels einer Pumpe wird ein Luftstrom in die Vorrichtung gesaugt. Am Gaseingang der Vorrichtung installierte Filter (einmal Zinkgranulat und zum anderen ein Aerosolfilter) entfernen alle stark sauer reagierenden Verbindungen und natürlich auftretende Aerosole aus dem Luftstrom. Dann wird der Luftstrom in einen Hauptstrom, der direkt zur Ionisationskammer geleitet wird, und einen Teilstrom, in den SO₂ mittels Permeation durch eine Polyethylenfolie dosiert wird, aufgeteilt. Der Teilstrom mit dem zudosierten SO₂ wird vor der Ionisationskammer wieder mit dem Hauptstrom vereinigt. Das Amin wird in die Ionisationskammer dosiert, so daß die Bildung der nachzuweisenden Aerosole direkt in der Kammer erfolgt. Die Konzentrationen des Amins betragen 50 ppm (Sättigung der Aerosolausbeute), die des SO₂ rund 3,5 ppm.

Mit der beschriebenen Vorrichtung können weniger als 5 ppb Diboran bei einem Meßbereich von 0 bis 500 ppb gemessen werden. Die Meßzeit ist gering (≥ 1 Minute), der Wartungsaufwand niedrig.

**Ansprüche**

1. Verfahren zur Bestimmung von Diboran in Luft auf der Grundlage der Aerosolionisationsgasanalyse mit Amin, dadurch gekennzeichnet, daß dem Luftstrom zusätzlich zum Amin ein Reagens zugesetzt wird, das nicht mit dem Amin, aber mit dem Diboran zu einer mit dem Amin Aerosole bildenden Verbindung reagiert.

2. Verfahren nach Punkt 1, dadurch gekennzeichnet, daß die Konzentration des Reagens' so gewählt wird, daß dadurch der größere Meßeffekt, verglichen mit dem durch die direkte Reaktion des Diborans mit dem Amin bewirkten Meßeffekt, entsteht.

3. Verfahren nach Punkt 1 und 2, dadurch gekennzeichnet, daß Schwefeldioxid in Konzentration ≥ 3,5 ppm zugesetzt wird.

4. Verfahren nach Punkt 1 und 3, dadurch gekennzeichnet, daß das Schwefeldioxid durch Zersetzung einer wäßrigen Na₂S₂O₅-Lösung gewonnen und durch Permeation in den Luftstrom dosiert wird.

5. Verfahren nach Punkt 1, dadurch gekennzeichnet, daß vor dem Zusatz von Reagens und Amin alle sauer reagierenden Verbindungen mittels Zinkgranulat aus dem Luftstrom entfernt werden.